Europäisches Patentamt

European Patent Office

Office européen des brevets

(19)

(11) **EP 1 009 393 B1**

(12) **EUROPÄISCHE PATENTSCHRIFT**

(45) Veröffentlichungstag und Bekanntmachung des Hinweises auf die Patenterteilung:
**03.04.2002 Patentblatt 2002/14**

(21) Anmeldenummer: **98948853.1**

(22) Anmeldetag: **21.08.1998**

(51) Int Cl.$^7$: **A61K 9/70**

(86) Internationale Anmeldenummer:
**PCT/EP98/05321**

(87) Internationale Veröffentlichungsnummer:
**WO 99/12529 (18.03.1999 Gazette 1999/11)**

(54) **TRANSDERMALES THERAPEUTISCHES SYSTEM MIT HAFTKLEBENDER RESERVOIRSCHICHT UND UNIDIREKTIONAL ELASTISCHER RÜCKSCHICHT**

TRANSDERMAL THERAPEUTIC SYSTEM COMPRISING A RESERVOIR-TYPE PRESSURE-SENSITIVE ADHESIVE LAYER AND A BACK LAYER WITH UNI-DIRECTIONAL RESILIENCE

SYSTEME THERAPEUTIQUE TRANSDERMIQUE A COUCHE-RESERVOIR AUTOCOLLANTE ET A COUCHE ENVERS A ELASTICITE UNIDIRECTIONNELLE

(84) Benannte Vertragsstaaten:
**AT BE CH DE DK ES FI FR GB GR IE IT LI LU NL PT SE**
Benannte Erstreckungsstaaten:
**SI**

(30) Priorität: **05.09.1997 DE 19738855**

(43) Veröffentlichungstag der Anmeldung:
**21.06.2000 Patentblatt 2000/25**

(73) Patentinhaber: **LTS Lohmann Therapie-Systeme AG**
**56626 Andernach (DE)**

(72) Erfinder:
• **HILLE, Thomas**
  **D-56567 Neuwied (DE)**
• **DEURER, Lothar**
  **D-56077 Koblenz (DE)**

(74) Vertreter: **Flaccus, Rolf-Dieter, Dr.**
**Patentanwalt**
**Bussardweg 10**
**50389 Wesseling (DE)**

(56) Entgegenhaltungen:
**WO-A-92/17237        DE-A- 4 333 595**
**DE-A- 4 423 850**

## Beschreibung

Die Erfindung betrifft ein transdermales therapeutisches System, insbesondere ein wirkstoffhaltiges Pflaster, umfassend eine wiederablösbare Schutzschicht, eine haftklebende Reservoirschicht sowie eine - gegebenenfalls haftkleberbeschichtete - Rückschicht. Außerdem betrifft die Erfindung ein Verfahren zur Herstellung eines derartigen transdermalen therapeutischen Systems [nachfolgend auch TTS abgekürzt] sowie die Verwendung eines solchen.

Ein TTS ist eine auf die Haut aufzubringende Applikationsform für über die Haut abzugebende Wirkstoffe, die das Aussehen traditioneller Pflaster aufweist. Es ist von einem topischen wirkstoffhaltigen Pflaster - beispielsweise einem Rheumapflaster oder einem Hühneraugenpflaster - zu unterscheiden. Ein solches TTS kann einen oder mehrere Wirkstoffe enthalten, die in vorherbestimmter Rate kontinuierlich über einen festgelegten Zeitraum am Applikationsort abgegeben werden ("Heilmann, Klaus: Therapeutische Systeme - Konzept und Realisation programmierter Arzneiverabreichung", 4. Auflage, 1984, Ferdinand-Enke-Verlag, Stuttgart). Üblicherweise beträgt der vorstehend genannte festgelegte Zeitraum 24 Stunden. Für die Behandlung von chronischen Erkrankungen ist jedoch die längerdauernde Gabe von Arzneistoffen erforderlich. Es kann daher sinnvoll sein, ein TTS auch über einen längeren Zeitraum als 24 Stunden zu applizieren, da dies eher zu konstanten Plasmaspiegeln führt.

Ein typisches derartiges transdermales therapeutisches System in Form eines Pflasters ist aus der EP-B 0 430 019 bekannt. Dieses weist eine wirkstoffundurchlässige Rückschicht, eine haftklebende Reservoirschicht und eine wiederablösbare Schutzschicht auf. Die wirkstoffundurchlässige Rückschicht kann aus flexiblem oder nicht flexiblem Material bestehen.

Als Substanzen, die zur Herstellung derartiger Materialien verwendet werden, sind Polymerfolien oder Metallfolien oder auch ein Verbundstoff aus einer mit Aluminium bedampften Folie erwähnt. Werden derartige TTS länger auf der Haut getragen, wie dies - wie vorstehend erwähnt - zur Behandlung von insbesondere chronischen Erkrankungen notwendig ist, so stellt sich, bedingt durch die relative Starrheit des TTS, auf der Haut innerhalb kurzer Zeit ein ausgeprägtes Fremdkörpergefühl ein. Dies ist für den Anwender äußerst unangenehm.

Eine andere Ausführungsform eines derartigen TTS ist in der US-A 5,246,705 beschrieben. Das dort beschriebene transdermale System weist eine elastomere Rückschicht auf, die eine definierte Dampfdurchlässigkeit im Bereich von 0,1 bis 20 g/m$^2$/hr sowie einen Young-Modul im Bereich von etwa 10$^4$ bis 10$^9$ dyne/cm$^2$ aufweist. Besonders bevorzugte Materialien für die elastomere Rückschicht sind beispielsweise A-B-A Blockcopolymere, wobei es sich bei den A-Blöcken um Styrol und bei den B-Blöcken um gesättigte Kohlenwasserstoff-Copolymere wie etwa Ethylen-Butylen-, Ethylen-Propylen-Copolymere u. ä. handelt. Auch beim Tragen der transdermalen therapeutischen Systeme gemäß der genannten US-A 5,246,705 für längere Zeit auf der Haut läßt sich das beschriebene Fremdkörpergefühl nicht vermeiden.

Aus der US-A 4,780,168 ist eine streifenförmige Wundbandage zum Versiegeln von Wunden bekannt, die aus einem gewebten oder nicht-gewebten Material auf der Basis eines Polymers hergestellt ist, wobei dieses Material eine planare Streckungscharakteristik im Bereich von 0,5 bis 110 [pounds/ inch] aufweist. Materialien mit einer derartigen Dehnbarkeit sind jedoch nicht ohne weiteres als Materialien für Rückschichten von transdermalen therapeutischen Systemen geeignet. Entweder ihre Dehnbarkeit ist zu gering, dann stellt sich beim längeren Tragen auf der Haut das vorstehend beschriebene, unangenehme Fremdkörpergefühl ein; sind sie aber zu sehr dehnbar, dann tritt bei der Herstellung von transdermalen therapeutischen Systemen der sog. "Schüsselbildungs"-Effekt auf, der nachfolgend erläutert wird.

Beim Herstellen des Laminats, aus dem die einzelnen wirkstoffhaltigen Pflaster gestanzt werden, gerät das Material für die Rückschicht unter Zugspannung und die sich ergebende elastische Rückstellkraft führt dazu, daß beim Stanzen der Pflaster die einander gegenüberliegenden Enden jeweils hochgebogen werden. Dieser Effekt führt bei der Fertigung wegen des dadurch bedingten Ausschusses zu hohen Kosten, verbunden mit unnötiger Umweltbelastung.

Abgesehen von den erwähnten Nachteilen ist ein Material für die Rückschicht einer Wundbandage auch aus anderen Gründen, wie der erforderlichen Wirkstoffundurchlässigkeit, nicht für ein TTS geeignet.

Aufgabe der Erfindung ist es deshalb, ein transdermales therapeutisches System bereitzustellen, das eine wiederablösbare Schutzschicht, eine haftklebende Reservoirschicht und eine gegebenenfalls haftkleberbeschichtete Rückschicht umfaßt, das die vorstehend genannten Nachteile vermeidet. Insbesondere soll sich bei längerem Tragen, auch über einige Tage bis zu etwa 1 bis 2 Wochen, kein Fremdkörpergefühl auf der Haut einstellen. Außerdem soll bei der Herstellung des TTS der sog. "Schüsselbildungs"-Effekt vermieden werden, was eine rationelle und kostengünstige Produktion sicherstellt.

Erfindungsgemäß wird die Aufgabe durch ein transdermales therapeutisches System, insbesondere ein wirkstoffhaltiges Pflaster, gelöst, das eine wieder-ablösbare Schutzschicht, eine haftklebende Reservoirschicht und eine gegebenenfalls haftkleberbeschichtete Rückschicht umfaßt, wobei die Rückschicht ein unidirektional, insbesondere längselastisches Material mit einer Elastizität von mindestens 20 % aufweist.

Bevorzugte Ausführungsformen des erfindungsgemäßen TTS sind Gegenstand der Unteransprüche.

Erfindungsgemäß weist das TTS neben einer wiederablösbaren Schutzschicht und einer haftklebenden Reservoirschicht auch eine wahlweise ebenfalls haftkleberbeschichtete Rückschicht auf, die eine speziell definierte unidirektionale Elastizität zeigt. Die Elastizität wird bei dem erfindungsgemäßen TTS gemäß den üblicherweise bei Elastizitätsprüfungen verwendeten DIN-Normen 60 000 und 61 632 (April 1985) bestimmt. Diese DIN-Normen gelten zwar ursprünglich für Idealbinden; die für die Prüfung der Elastizität dabei verwendete Horizontal-Kraftdehnungsanlage ist aber auch bei anderen Materialien analog einsetzbar. Erfindungsgemäß ist bei dem TTS die Rückschicht in nur einer Richtung, d. h. in Längs- oder Querrichtung elastisch. Bezogen auf die Längsachse des TTS ist die Querachse, die im rechten Winkel dazu liegende Achse. Bei einem kreisförmigen TTS sind Längs- und Querachse naturgemäß längenmäßig identisch. Insbesondere ist das erfindungsgemäß verwendete Material der Rückschicht unidirektional längselastisch.

In der anderen Richtung ist die Rückschicht nicht elastisch. "Nicht-elastisch" bedeutet, daß bei Testung per Hand keine Elastizität feststellbar ist. Bei Messung gemäß DIN 61 632 liegt dann die Elastizität unterhalb von 20 %. Erfindungsgemäß liegt also die Elastizität in einer - nämlich der elastischen Richtung - oberhalb von 20 %.

Da beim Herstellen des Pflasters dasselbe aus einem Laminat ausgestanzt wird, wäre es prinzipiell auch denkbar, daß das TTS in einem Winkel zur Längsrichtung des Pflasters "unidirektional" elastisch ist. Eine derartige Schrägelastizität ist jedoch das Ergebnis einer Superposition von Elastizität in Quer- und in Längsrichtung.

Bevorzugt wird bei dem erfindungsgemäßen TTS ein unidirektional elastisches Material für die Rückschicht verwendet, dessen Elastizität kleiner als 150 % ist. Bei einer stärker bevorzugten Ausführungsform liegt die Elastizität im Bereich von 20 bis 80 %, besonders bevorzugt im Bereich zwischen 40 bis 70 %. Am allerbevorzugtesten und demzufolge für die Lösung der der Erfindung zugrundeliegenden Aufgabe am vorteilhaftesten ist ein Material für die Rückschicht, dessen Elastizität - immer gemessen gemäß DIN 61 632 - im Bereich zwischen 44 und 56 % liegt.

Bevorzugte Materialien für die unidirektional elastische Rückschicht sind mikrobiell nicht abbaubare Stoffe. Das Material solite zu mehr als 90 %, bevorzugt zu mehr als 99 %, mikrobiell nicht abbaubar sein. Die Abbaubarkeit kann mit üblichen, dem Fachmann vertrauten Methoden gemessen werden. Die geringe Abbaubarkeit ist besonders wichtig bei TTS, die länger auf der Haut getragen werden sollen. Denn bedingt durch die Transpiration der Haut entsteht unmittelbar unter der durch das TTS abgedeckten Hautpartie ein Mikroklima, in dem Bakterien, Pilze, Sporen etc. prächtig gedeihen. Deshalb ist eine geringe mikrobielle Abbaubarkeit, insbesondere bei längeren Tragezeiten, äußerst vorteilhaft. Bei dem Material der Rückschicht kann es sich um ein Gewebe, ein Vlies oder eine Folie handeln. Umfaßt die Rückschicht ein Polymer, so ist dieses vorteilhafterweise aus Polyethylen, Polypropylen oder Polyestern, insbesondere Polyalkylenterephthalaten ausgewählt.

Beispielhaft seien einige polymere Materialien für die Rückschicht genannt. Günstige polymere Materialien, die das vorstehende Erfordernis der geringen mikrobiellen Abbaubarkeit erfüllen, sind Polyterephthalsäurediester, die durch die Umsetzung eines Ausgangsstoffs, ausgewählt aus Ethylenglykol, 1,4-Butandiol, 1,4-Dihydroxymethylcyclohexan, Terephthalsäure, Isophthalsäure, Adipinsäure, Azelainsäure, Sebacinsäure, Terephthalsäuredimethylester, Azelainsäuredimethylester, Sebacinsäuredimethylester, Bisphenol-A-Diglycidether, n-Decandicarbonsäure-1.10, Polyethylenglykol und Polybutylenglykol erhältlich sind.

Bei dem erfindungsgemäßen transdermalen therapeutischen System kann zwischen Rückschicht und Reservoirschicht ebenfalls noch eine weitere Trennschicht, z. B. zur Vermeidung der Wirkstoffdurchlässigkeit, angeordnet sein. In diesem Fall ist die Rückschicht auf der der Haut zugewandten Seite bzw. die Trennschicht auf der distalen Seite ebenfalls haftkleberbeschichtet.

Für die Wirksamkeit eines erfindungsgemäßen TTS ist es günstig, wenn die Rückschicht das Reservoir allseitig überragt. Dies hat den Vorteil, daß es in seitlicher Richtung nicht zu Wirkstoffverlusten kommt. Außerdem kann das erfindungsgemäße TTS dann besonders kostengünstig hergestellt werden, da die wirkstoffhaltigen Abschnitte separat gestanzt werden können. So werden teure und umweltschädliche, wirkstoffhaltige Abfallreststücke vermieden. Die Rückschicht des TTS weist eine Wasserdampfpermeabilität von mindestens 0,1 $g/m^2/h$, bevorzugt von 1 bis 20 $g/m^2/h$ auf.

Im Falle der Verwendung eines Vlieses oder eines Gewebes oder auch einer porösen Folie liegt die Porosität im Bereich von 10 % bis 50 %. Dabei bedeutet "Porosität" den Flächenanteil von Poren mit einer Fläche von $\leq 400$ $\mu m^2$ an der jeweiligen Bezugsfläche. Diese relative Porenfläche kann durch Messung und Zählen der Poren einer beliebigen ungedehnten Bezugsfläche unter dem Mikroskop oder einem Fadenzähler ermittelt werden.

Falls für das erfindungsgemäße transdermale therapeutische System (TTS) ein Gewebe verwendet wird, so weist die Rückschicht eine Kettfadenzahl im Bereich von 300 - 350, bevorzugt im Bereich von 310 - 330, und/oder eine Schußfadenzahl im Bereich von 100 bis 140, bevorzugt im Bereich von 120 bis 130, jeweils gemessen je 10 cm ungedehntes Gewebe, auf.

Die haftklebende Reservoirschicht des erfindungsgemäßen transdermalen therapeutischen Systems enthält mindestens einen Wirkstoff. Bevorzugt ist dieser ausgewählt aus der Gruppe der Psychopharmaka, Analgetika und Hormone. Als Hormon kommt insbesondere Estradiol in Betracht, als Analgetikum Buprenorphin. Bei dem Psychopharmakon handelt es sich bevorzugt um ein Parasympathomimetikum.

In Frage kommende Parasympathomimetika sind insbesondere:

1. Cholinester, z. B. Acetylcholin, Bethanechol, Carbachol oder Methacholin,
2. Alkaloide, z. B. Arecolin und seine Derivate, Pilocarpin,
3. Hemmstoffe der Cholinesterase, z. B. Demecariumbromid, Distigminbromid, Neostigmin, Physostigmin, Pyridostigminbromid, Galanthamin.

Natürlich können diese Stoffe auch in Kombination miteinander verwendet werden. Der Gehalt an Wirkstoff wird insbesondere dabei so eingestellt, daß es beim Abnehmen des Pflasters zu einem sog. "Abreißeffekt" kommt. Dieser Effekt wird nachfolgend näher erläutert:

Enthält ein TTS eine begrenzt wasserdampfdurchlässige Rückschicht, z. B. eine PET-Folie, kann während des Tragens des TTS die Haut am Applikationsort keinen Wasserdampf abatmen. Dieses Wasser wird in die Haut eingelagert. Am Applikationsort ist deshalb der Wassergehalt höher als physiobiologischerweise bedingt. Ein nur schwer von der Haut resorbierbarer Stoff (wie z.B. Buprenorphin) wird in ein Depot in der Haut eingelagert werden. Beim Abreißen des TTS atmet die Haut schlagartig Wasserdampf ab.

Durch Entfernen dieses Wassers steigt plötzlich die Konzentration des Arzneistoffs über die Sättigungskonzentration an, da Lösemittel entfernt wird. Ein stabiler Zustand wird dadurch erreicht, daß sich das Hautdepot rasch entleert. Es kommt daher durch das Abreißen des TTS zu einem raschen Konzentrationsanstieg des Wirkstoffs im Plasma. Zur besseren Wirkstoffausnutzung ist die Ausnutzung des Abreißeffekt bevorzugt. Erfindungsgemäß wird daher die Konzentration des Wirkstoffs so eingestellt, daß der vorstehend genannte Abreißeffekt zum Tragen kommt.

Die absolute Höhe des Gehalts an Wirkstoff zur Erzielung des Abreißeffekts läßt sich nicht exakt allgemein gültig definieren. Sie schwankt von Wirkstoff zu Wirkstoff und hängt auch von der Ausführungsform des TTS ab. Die Einstellung kann jedoch vom Fachmann mittels Routineversuchen ohne unzumutbaren Aufwand vorgenommen werden. Im Falle des Buprenorphins liegt der Gehalt etwa bei 5 - 15 Gew.-%.

Die haftklebende Reservoirschicht kann auch ein wasseraufnehmendes Polymer enthalten. Bei einer bevorzugten Ausführungsform handelt es sich bei dem wasseraufnehmenden Polymer um ein Polyvinylpyrrolidon. Das Polyvinylporrolidon weist bevorzugterweise ein Molekulargewicht im Bereich von $1 \times 10^3$ bis $2 \times 10^6$ auf. Zu derartigen Polyvinylpyrrolidonen gehört Koliidon® .

Für besondere Zwecke, beispielsweise für die Verwendung in Krankenhäusern mit vielen Patienten oder für die Verwendung in Doppelblindstudien, wo wirkstoffhaltige TTS mit Placebo-TTS verglichen werden, ist es außerdem bevorzugt, wenn die nach außen - also von der Haut weg - gerichtete Seite des TTS in der Rückschicht ein von der übrigen Fläche abgesetztes Markierungs-Steuerungselement aufweist.

Dieses Element kann sich in der Struktur oder übrigen Eigenschaften, wie Elastizität oder Porosität, von dem übrigen Teil der Rückschicht unterscheiden. Durch ein derartiges Markierungs-Steuerungselement können die Eigenschaften der Rückschicht differentiell eingestellt werden. Beispielsweise kann die Elastizität eines solchen Elementes größer sein als die Elastizität des übrigen Teils der Rückschicht. Falls ein derartiges Markierungs-Steuerungselement in einem Teil der Rückschicht speziell eingebracht ist, so liegt seine relative Elastizität - für den Fall, daß dieses gewünscht wird - vorzugsweise in einem Bereich, der um 20 % unterhalb oder um 20 % oberhalb der Elastizität des übrigen Teils der Rückschicht liegt.

Das Markierungs-Steuerungselement kann auch dazu dienen, die einzelnen TTS bezüglich ihres Wirkstoffgehalts voneinander zu unterscheiden. Dies erfolgt bevorzugt durch eine farbige Markierung, beispielsweise durch einen farbigen Faden oder Streifen. Das ist besonders vorteilhaft, wenn das TTS an einem Ort, beispielsweise einem Krankenhaus mit vielen Patienten, in großer Menge in unterschiedlichen Dosierungen bereitgehalten werden muß.

Das erfindungsgemäße transdermale therapeutische System eignet sich insbesondere zur Anwendung als Mehrtagespflaster wegen seiner auf diesen Bedarf besonders zugeschnittenen Rückschicht. Insofern kommt es insbesondere zur Behandlung von chronischen Schmerzen oder aber auch zur Behandlung von Drogenabhängigkeit in Frage.

Das erfindungsgemäße TTS wird mittels üblicher Verfahren hergestellt. Ein derartiges Verfahren umfaßt i. allg. die Schritte, daß aus einem vorgelegten bandförmigen Laminat, umfassend die erfindungsgemäße unidirektional elastische Rückschicht, eine Wirkstoffschicht sowie eine wiederablösbare Schutzschicht, durch Stanzen die einzelnen TTS hergestellt werden.

Bei einem besonders bevorzugten Verfahren zur Herstellung des erfindungsgemäßen TTS sind die vorstehend genannten Schritte so modifiziert, daß bei einem vorgelegten bandförmigen Laminat mit einer gegebenenfalls haftklebenden, unidirektional elastischen Rückschicht und einer wiederablösbaren Schutzschicht in Längsrichtung hintereinander haftklebende Wirkstoffreservoirabschnitte eingelegt werden, die Rückschicht durch Stanzen und anschließend in den Zwischenräumen zwischen den Wirkstoffreservoirabschnitten die Schutzschicht durchtrennt wird. Dieses spezielle Verfahren hat den Vorteil, daß es unter ökonomischen und auch unter ökologischen Gesichtspunkten sehr vorteilhaft ist. Durch das separate Einlegen der Wirkstoffreservoirabschnitte wird nämlich vermieden; daß Abfall mit - in der Regel sehr teurem - Wirkstoff entsteht und kostspielig entsorgt werden muß. Ein analoges Verfahren ist in der DE-B 41 10 027 beschrieben, auf die diesbezüglich ausdrücklich Bezug genommen wird.

Die Erfindung wird nachfolgend anhand einer Zeichnung und eines Ausführungsbeispiels erläutert. Es zeigen die Figuren:

Fig. 1 das erfindungsgemäße TTS in Draufsicht;
Fig. 2 das TTS gemäß Fig. 1, geschnitten längs der Stellen II - II.

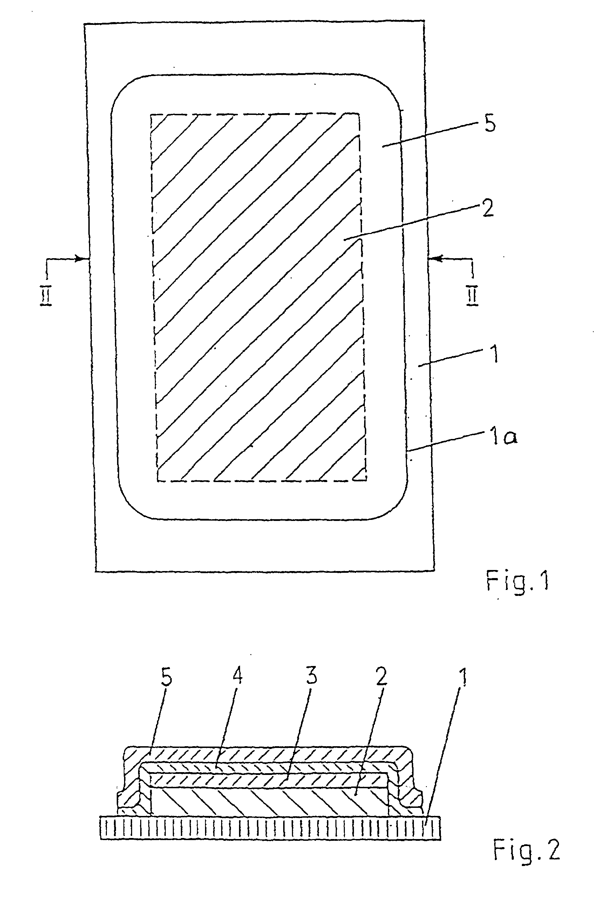

Fig. 1 zeigt schematisch ein erfindungsgemäßes TTS in Draufsicht. Auf der wiederablösbaren, im vorliegenden Falle rechteckigen, Schutzschicht (1) liegt die mit wirkstofffreiem Haftkleber beschichtete Rückschicht (5) auf. Sie weist die Form eines Rechtecks mit abgerundeten Ecken auf. Die Stanzlinie (1a) umreißt die Form der Rückschicht (5). Sie verläuft außerhalb des Laminats aus Reservoir (2) sowie wahlweiser Sperr- oder Trennschicht (3). Durch den Verlauf der Stanzlinie wird beim Ausstanzen Wirkstoffverlust vermieden. Innerhalb der Rückschicht (5) sind die Konturen aus Reservoir (2) sowie der wahlweisen Sperrschicht (3) erkennbar.

Bei dem dargestellten TTS mit unidirektional elastischer Rückschicht (5) überragt dieselbe das vorstehend erwähnte Laminat allseitig. Das Reservoir hat bevorzugt eine rechteckige Form. Die rechteckige Form ist deshalb bevorzugt, da so beim Schneiden des Reservoirs Wirkstoffverluste vermeidbar sind.

Fig. 2 ist ein Querschnitt gemäß II - II der Fig. 1. Der Deutlichkeit halber sind die Schichtdicken übertrieben dargestellt. Das TTS weist das Reservoir (2), die abziehbare Schutzschicht (1) sowie die wahlweise vorhandene Sperrschicht (3) und eine weitere - im Falle des Vorhandenseins einer Sperrschicht (3) notwendige - wirkstofffreie Haftkleberschicht (4) auf. Bei dieser dargestellten Ausführungsform wird das erwähnte Laminat von der Rückschicht (5) und der wirkstofffreien Haftkleberschicht (4) allseitig überragt.

**Beispiel**

Zur Herstellung der erfindungsgemäßen unidirektional elastischen Rückschicht wurde ein Polyestergewebe mit den folgenden Merkmalen mittels der dem Fachmann bekannten Techniken hergestellt.

| PRÜFMERKMALE | EINHEIT | Soll | MIN | MAX | $\bar{X}$ |
|---|---|---|---|---|---|
| **WARENBREITE** | mm | 1500 | 1300 | 1390 | 1360 |
| **FLÄCHENGEWICHT** (ungedehnt) (DIN 53854 + DIN 53884) | g/m$^2$ | 100 | 95 | 103 | 100 |
| **DEHNUNG** (längs) | % | - | - | - | - |
| (quer) | % | 50 | 46 | 52 | 48 |
| (DIN 61632) | | | | | |
| **KETTFADENZAHL** 10 cm ungedehnt | | 320 | 310 | 330 | 324 |
| **SCHUßFADENZAHL** 10 cm ungedehnt | | 125 | 124 | 126 | 124 |

Außerdem wurden

| 49,175 kg | Durotak Typ 387-2054 (48,3 %-ige Lösung) |
|---|---|
| 4,450 kg | geschmolzene Lävulinsäure und |
| 6,675 kg | Oleyloleat |

unter Rühren homogenisiert. Anschließend wurden portionsweise 4,450 kg Kollidon 90F zugesetzt. Nach Verdünnen mit 6,800 kg Ethanol wurde 5 Stunden bei 170 -190 Ulmin gerührt. Dann wurden 4,450 kg Buprenorphinbase, angeschlämmt in 4,500 kg Ethylacetat, hinzugegeben. Mit 4,500 kg Ethylacetat wurde verdünnt.

Es wurde ca. 7 Stunden lang mit 170 Ulmin gerührt. Anschließend erfolgte eine Prüfung auf Homogenität. War die Masse homogen, wurde sie bei abgeschaltetem Rührer entgast.

Nach erfolgter Homogenisierung wurde die Klebermasse auf eine silikonisierte Polyesterfolie aufgestrichen. Durch Trocknen bei üblicherweise 35 °C bis 80 °C wurden die organischen Lösungsmittel entfernt. Anschließend wurde das Laminat aus silikonisierter Polyesterfolie und buprenorphinhaltiger Haftkleberschicht mit einer 23 um dicken zweiten Polyesterfolie abgedeckt.

Von dem so erhaltenen wirkstoffhaltigen Laminat wurde die silikonisierte Polyesterfolie entfernt. Danach wurden 50 cm$^2$ große Rechtecke ausgestanzt und mit der klebenden Seite in Abständen von 3 cm auf die silikonisierte Seite einer weiteren 100 μm Polyesterschutzfolie aufgelegt. Über diese Reservoirabschnitte wurde das - in diesem Falle ebenfalls haftkleberbeschichtete - unidirektional elastische Polyestergewebe gelegt. Anschließend wurden daraus längselastische Einzelpflaster ausgestanzt. Mit diesem erfindungsgemäßen TTS wurde an n=10 Probanden ein Tragetest durchgeführt.

**Vergleichsbeispiel 1**

Anstelle des erfindungsgemäßen unidirektional elastischen Polyestergewebes wurde bei diesem Beispiel ein bidirektional elastisches Polyestergewebe verwendet. Die Dehnbarkeit dieses Gewebes (Längs- und Querdehnung) betrug 30 %, gemessen nach DIN 61 632. Das Flächengewicht betrug 109 g/m$^2$. Bei dem Material handelte es sich um ein Polyethylenterephthalat. Im übrigen stimmten die gemäß diesem Vergleichsbeispiel hergestellten TTS mit denjenigen des erfindungsgemäßen Beispiels überein.

Unter Verwendung der TTS gemäß diesem Vergleichsbeispiel wurde ebenfalls an n=10 Probanden ein Tragetest durchgeführt.

**Vergleichsbeispiel 2**

Entsprechend Beispiel 1 und Vergleichsbeispiel 1 wurden TTS hergestellt, nur daß dabei statt einer unidirektional elastischen Rückschicht bzw. einer bidirektional elastischen Rückschicht eine haftkleberbeschichtete starre Polyesterfolie (15 μm Dicke) [Hostaphan® RN 15, Hoechst AG) eingesetzt wurde.

Mit den so erhaltenen TTS wurde auch in diesem Fall wieder an n=10 Probanden ein Tragetest durchgeführt

**Auswertung**

Der Vergleichstragetest der TTS gemäß Beispiel 1, Vergleichsbeispiel 1 und Vergleichsbeispiel 2 ergab folgendes Ergebnis:

Bei der Verwendung von Polyesterfolie als Rückschicht (Vergleichsbeispiel 2) trat bereits am ersten Tag ein Fremdkörpergefühl auf. Am zweiten Tag erfolgte Faltenbildung und - beginnend mit dem dritten Tag - ein Ablösen des TTS. Das TTS gemäß Beispiel 1 sowie dasjenige gemäß Vergleichsbeispiel 1 wurde von allen 10 Probanden ohne Fremdkörpergefühl, ohne Beeinträchtigung der Klebkraft und auch ohne Hautreizungen über mindestens sieben Tage problemlos getragen. In bezug auf den Tragekomfort sind also das TTS gemäß Beispiel 1 sowie dasjenige gemäß Vergleichsbeispiel 1 etwa gleichwertig. Jedoch traten bei der Herstellung des TTS gemäß Vergleichsbeispiel 1 in einer Häufigkeit von mehr als 50 % Komplikationen bei der Herstellung auf, die vorwiegend auf den Schüsselbildungseffekt zurückzuführen waren.

**Patentansprüche**

1.   Transdermales therapeutisches System, insbesondere Pflaster, umfassend

   -   eine wiederablösbare Schutzschicht,
   -   eine haftklebende Reservoirschicht und
   -   eine gegebenenfalls haftkleberbeschichtete Rückschicht mit einem unidirektional, vorzugsweise längselastischen Material mit einer Elastizität von mindestens 20 %.

2.   Transdermales therapeutisches System nach Anspruch 1, wobei die Elastizität kleiner als 150 % ist.

3.   Transdermales therapeutisches System nach Anspruch 1 oder 2, wobei die Rückschicht das Resorvoir allseitig überragt.

4.   Transdermales therapeutisches System nach einem der vorhergehenden Ansprüche, wobei zwischen haftkleberbeschichteter Rückschicht und Reservoirschicht eine Trennschicht angeordnet ist.

5.   Transdermales therapeutisches System nach einem der einem der vorhergehenden Ansprüche, wobei das elastische Material eine Elastizität im Bereich von 20 - 80 %, besonders bevorzugt im Bereich von 40 - 70 %, am allerbevorzugtesten im Bereich von 44 - 56 % aufweist.

6. Transdermales therapeutisches System nach einem der vorhergehenden Ansprüche, wobei das Material der Rückschicht zu mehr als 90 %, bevorzugt mehr als 99 % mikrobiell nicht abbaubar ist.

7. Transdermales therapeutisches System nach einem der vorhergehenden Ansprüche, wobei es sich bei der Rückschicht um ein Gewebe, ein Vlies oder eine Folie handelt.

8. Transdermales therapeutisches System nach einem der vorhergehenden Ansprüche, wobei die Rückschicht im wesentlichen ein Material, ausgewählt aus der Gruppe der Polyethylene, Polypropylene und Polyester, insbesondere ausgewählt aus den Polyalkylenterephthalaten, enthält.

9. Transdermales therapeutisches System nach Anspruch 8, wobei das Material der Rückschicht ein Polyterephthalsäurediester, bevorzugt ein aus der Umsetzung eines Ausgangsstoffs, ausgewählt aus Ethylenglykol, 1,4-Butandiol, 1,4-Dihydroxymethylcyclohexan, Terephthalsäure, Isophthalsäure, Adipinsäure, Azelainsäure, Sebacinsäure, Terephthalsäuredimethylester, Azelainsäuredimethylester, Sebacinsäuredimethylester, Bisphenol-A-Diglycidether, n-Decandicarbonsäure-1.10, Polyethylenglykol und Polybutylenglykol, erhältlicher Polyterephthalsäurediolester ist.

10. Transdermales therapeutisches System nach einem der vorhergehenden Ansprüche, wobei die haftklebende Reservoirschicht mindestens einen Wirkstoff, bevorzugt ausgewählt aus der Gruppe der Psychopharmaka, Analgetika und Hormone, enthält.

11. Transdermales therapeutisches System nach Anspruch 10, wobei das Hormon Estradiol, das Analgetikum Buprenorphin und das Psychopharmakon ein Parasympathomimetikum ist.

12. Transdermales therapeutisches System nach einem der vorhergehenden Ansprüche, wobei die haftklebende Reservoirschicht ein wasseraufnehmendes Polymer enthält.

13. Transdermales therapeutisches System nach Anspruch 12, wobei das wasseraufnehmende Polymer ein Polyvinylpyrrolidon, bevorzugt eines mit einem Molekulargewicht im Bereich von $1 \times 10^3$ bis $2 \times 10^6$ ist.

14. Transdermales therapeutisches System nach einem der vorhergehenden Ansprüche, wobei die nach außen gerichtete Seite der Rückschicht ein von der übrigen Fläche abgesetztes Markierungs-Steuerungsselement aufweist.

15. Transdermales therapeutisches System nach Anspruch 14, wobei das Markierungs-Steuerungselement eine farbige Markierung, bevorzugt in Streifenform, oder ein farbiger Faden ist.

16. Transdermales therapeutisches System nach einem der Ansprüche 14 oder 15, wobei das Markierungs-Steuerungselement eine Elastizität aufweist, die im Bereich von -20 % bis +20 % liegt, bezogen auf die Elastizität des übrigen Teils der Rückschicht.

17. Transdermales therapeutisches System nach einem der vorherigen Ansprüche, wobei die Rückschicht eine Wasserdampfpermeabilität von mindestens 0,1 $g/m^2/h$, bevorzugt von 1 bis 20 $g/m^2/h$ aufweist.

18. Transdermales therapeutisches System nach einem der vorhergehenden Ansprüche, wobei das System einen Flächenanteil von Poren mit einer Größe $\leq 400 \ \mu m^2$ im Bereich von 10 % bis 50 % aufweist.

19. Transdermales therapeutisches System nach einem der vorherigen Ansprüche, wobei die Rückschicht eine Kettfadenzahl im Bereich von 300 bis 350, bevorzugt im Bereich von 310 bis 330, und/oder eine Schußfadenzahl im Bereich von 100 bis 140, bevorzugt im Bereich von 120 bis 130, jeweils je 10 cm ungedehntes Gewebe, aufweist.

20. Verfahren zur Herstellung des transdermalen therapeutischen Systems nach einem der Ansprüche 1 bis 19, umfassend die Schritte, daß

- bei einem vorgelegten bandförmigen Laminat mit einer gegebenenfalls haftklebenden, unidirektional elastischen Rückschicht und einer wiederablösbaren Schutzschicht in Längsrichtung hintereinander haftklebende Wirkstoffreservoirabschnitte eingelegt werden,
- die Rückschicht durch Stanzen durchtrennt wird,
- der Verschnitt der Rückschicht entfernt und

- anschließend in den Zwischenräumen zwischen den Wirkstoffreservoirabschnitten die Schutzschicht durchtrennt wird.

21. Transdermales therapeutisches System nach einem der Ansprüche 1 bis 19 zur Anwendung als Mehrtagespflaster, insbesondere zur Behandlung von Schmerzen oder Drogenabhängigkeit.

**Claims**

1. Transdermal therapeutic system, in particular a patch, comprising

   - a redetachable protective layer,
   - a pressure-sensitive adhesive reservoir layer and
   - a backing layer with or without a coating of pressure-sensitive adhesive and featuring a unidirectionally, preferably longitudinally, elastic material having an elasticity of at least 20%.

2. Transdermal therapeutic system according to Claim 1, wherein the elasticity is less than 150%.

3. Transdermal therapeutic system according to Claim 1 or 2, wherein the backing layer projects beyond the reservoir on all sides.

4. Transdermal therapeutic system according to one of the preceding claims, wherein a separating layer is arranged between the reservoir layer and the backing layer coated with pressure-sensitive adhesive.

5. Transdermal therapeutic system according to one of the preceding claims, wherein the elastic material has an elasticity in the range 20-80%, with particular preference in the range 40-70%, most preferably in the range 44-56%.

6. Transdermal therapeutic system according to one of the preceding claims, wherein the material of the backing layer is more than 90%, preferably more than 99%, microbially nondegradable.

7. Transdermal therapeutic system according to one of the preceding claims, wherein the backing layer is a woven fabric, a nonwoven fabric or a film.

8. Transdermal therapeutic system according to one of the preceding claims, wherein the backing layer essentially comprises a material selected from the group consisting of polyethylenes, polypropylenes and polyesters, selected in particular from the polyalkylene terephthalates.

9. Transdermal therapeutic system according to Claim 8, wherein the material of the backing layer is a polyterephthalic diester, preferably a polyterephthalic acid diol ester obtainable by the reaction of a starting material selected from ethylene glycol, 1,4-butanediol, 1,4-dihydroxymethylcyclohexane, terephthalic acid, isophthalic acid, adipic acid, azelaic acid, sebacic acid, dimethyl terephthalate, dimethyl azelate, dimethyl sebacate, bisphenol A diglycidyl ether, n-decane-1,10-dicarboxylic acid, polyethylene glycol and polybutylene glycol.

10. Transdermal therapeutic system according to one of the preceding claims, wherein the pressure-sensitive adhesive reservoir layer comprises at least one active substance selected preferably from the group consisting of psychopharmaceuticals, analgesics and hormones.

11. Transdermal therapeutic system according to Claim 10, wherein the hormone is oestradiol, the analgesic is buprenorphine and the psychopharmaceutical is a parasympathomimetic.

12. Transdermal therapeutic system according to one of the preceding claims, wherein the pressure-sensitive adhesive reservoir layer contains a water-absorbing polymer.

13. Transdermal therapeutic system according to Claim 12, wherein the water-absorbing polymer is a polyvinylpyrrolidone, preferably one having a molecular weight in the range from $1 \times 10^3$ to $2 \times 10^6$.

14. Transdermal therapeutic system according to one of the preceding claims, wherein the side of the backing layer which faces outwards has a marking/control element which is differentiated from the remaining area.

15. Transdermal therapeutic system according to Claim 14, where the marking/control element is a coloured marking, preferably in stripe form, or a coloured thread.

16. Transdermal therapeutic system according to one of Claims 14 and 15, wherein the marking/control element which has an elasticity in the range from -20% to +20% relative to the elasticity of the remaining portion of the backing layer.

17. Transdermal therapeutic system according to one of the previous claims, wherein the backing layer has a water vapour permeability of at least 0.1 g/m$^2$/h, preferably from 1 to 20 g/m$^2$/h.

18. Transdermal therapeutic system according to one of the preceding claims, wherein the areal proportion of pores having a size of $\leq$ 400 $\mu$m$^2$ is in the range from 10% to 50%.

19. Transdermal therapeutic system according to one of the previous claims, wherein the backing layer has a number of warp threads in the range from 300 to 350, preferably in the range from 310 to 330, and/or a number of weft threads in the range from 100 to 140, preferably in the range from 120 to 130, in each case per 10 cm of unextended fabric.

20. A process for producing the transdermal therapeutic system according to one of Claims 1 to 19, comprising the steps of

   - in a presupplied strip-like laminate having an optionally pressure-sensitive adhesive, unidirectionally elastic backing layer and a redetachable protective layer, inserting pressure-sensitive adhesive active substance reservoir sections in sequence in the longitudinal direction,
   - separating the backing layer by punching,
   - removing the unwanted cut portion of the backing layer and
   - then separating the protective layer in the spaces between the active substance reservoir sections.

21. Transdermal therapeutic system according to one of Claims 1 to 19 for use as a multi-day plaster, in particular for the treatment of pain or of drug dependency.

**Revendications**

1. Système thérapeutique transdermique, en particulier emplâtre, comprenant

   - une couche de protection pelliculable,
   - une couche auto-adhésive faisant office de réservoir,
   - une couche dorsale le cas échéant enduite d'un agent auto-adhésif comprenant une matière manifestant une élasticité unidirectionnelle, de préférence en direction longitudinale, dont l'élasticité représente au moins 20 %.

2. Système thérapeutique transdermique selon la revendication 1, dans lequel l'élasticité représente moins de 150 %.

3. Système thérapeutique transdermique selon la revendication 1 ou 2, dans lequel la couche dorsale recouvre le réservoir de toute part.

4. Système thérapeutique transdermique selon l'une quelconque des revendications précédentes, dans lequel on dispose une couche de séparation entre la couche dorsale enduite d'un agent auto-adhésif et la couche faisant office de réservoir.

5. Système thérapeutique transdermique selon l'une quelconque des revendications précédentes, dans lequel la matière élastique présente une élasticité dans le domaine de 20 à 80 %, de manière particulièrement préférée dans le domaine de 40 à 70 %, de manière tout à fait préférée dans le domaine de 44 à 56 %.

6. Système thérapeutique transdermique selon l'une quelconque des revendications précédentes, dans lequel la matière de la couche dorsale manifeste une inaptitude à la décomposition microbienne jusqu'à concurrence d'une valeur supérieure à 90 %, de préférence supérieure à 99 %.

**7.** Système thérapeutique transdermique selon l'une quelconque des revendications précédentes, dans lequel, en ce qui concerne la couche dorsale, il s'agit d'un tissu, d'un non-tissé ou d'une feuille.

**8.** Système thérapeutique transdermique selon l'une quelconque des revendications précédentes, dans lequel la couche dorsale contient essentiellement une matière choisie parmi le groupe comprenant des polyéthylènes, des polypropylènes et des polyesters, en particulier choisi parmi les polyalkylènetéréphtalates.

**9.** Système thérapeutique transdermique selon la revendication 8, dans lequel la matière de la couche dorsale est un diester polytéréphtalique, de préférence un esterdiol de l'acide polytéréphtalique obtenu par la mise en réaction d'une substance de départ choisie parmi le groupe comprenant l'éthylèneglycol, le 1,4-butanediol, le 1,4-dihydroxyméthyl-cyclohexane, l'acide téréphtalique, l'acide isophtalique, l'acide adipique, l'acide azélaïque, l'acide sébacique, l'ester diméthylique de l'acide téréphtalique, l'ester diméthylique de l'acide azélaïque, l'ester diméthylique de l'acide sébacique, l'éther diglycidylique de Bisphénol-A, l'acide n-décanedicarboxylique-1,10, le polyéthylèneglycol et le polybutylèneglycol.

**10.** Système thérapeutique transdermique selon l'une quelconque des revendications précédentes, dans lequel la couche auto-adhésive faisant office de réservoir contient au moins une substance active, de préférence choisie parmi le groupe comprenant des psychotropes, des analgésiques et des hormones.

**11.** Système thérapeutique transdermique selon la revendication 10, dans lequel l'hormone est l'oestradiol, l'analgésique est la buprénorphine et le psychotrope est un médicament parasympathomimétique.

**12.** Système thérapeutique transdermique selon l'une quelconque des revendications précédentes, dans lequel la couche auto-adhésive faisant office de réservoir contient un polymère hydrophile.

**13.** Système thérapeutique transdermique selon la revendication 12, dans lequel le polymère hydrophile est une polyvinylpyrrolidone, de préférence une polyvinylpyrrolidone dont le poids moléculaire se situe dans le domaine de $1 \times 10^3$ à $2 \times 10^6$.

**14.** Système thérapeutique transdermique selon l'une quelconque des revendications précédentes, dans lequel le côté de la couche dorsale tourné vers l'extérieur présente un élément de marquage-mise en oeuvre contrastant de l'autre surface.

**15.** Système thérapeutique transdermique selon la revendication 14, dans lequel l'élément de marquage-mise en oeuvre est un marqueur coloré, de préférence sous forme d'une bande, ou un fil coloré.

**16.** Système thérapeutique transdermique selon l'une quelconque des revendications 14 ou 15, dans lequel l'élément de marquage-mise en oeuvre présente une élasticité qui se situe dans le domaine de -20 % à +20 %, rapportée à l'élasticité de la partie restante de la couche dorsale.

**17.** Système thérapeutique transdermique selon l'une quelconque des revendications précédentes, dans lequel la couche dorsale présente une perméabilité à la vapeur d'eau qui s'élève à au moins 0,1 g/m$^2$/h, de préférence de 1 à 20 g/m$^2$/h.

**18.** Système thérapeutique transdermique selon l'une quelconque des revendications précédentes, dans lequel le système présente une fraction superficielle occupée par les pores possédant une dimension $\leq 400$ µm$^2$ dans le domaine de 10 % à 50 %.

**19.** Système thérapeutique transdermique selon l'une quelconque des revendications précédentes, dans lequel la couche dorsale présente un nombre de fils de chaîne dans le domaine de 300 à 350, de préférence de 310 à 330 et/ou un nombre de fils de trame dans le domaine de 100 à 140, de préférence dans le domaine de 120 à 130, respectivement tous les 10 cm du tissu à l'état non étiré.

**20.** Procédé pour la fabrication d'un système thérapeutique transdermique selon l'une quelconque des revendications 1 à 19, comprenant les étapes consistant à :

- incorporer dans un stratifié en forme de bande déposé au préalable, comprenant une couche dorsale élastique unidirectionnelle, le cas échéant auto-adhésive, et une couche de protection pelliculable, de manière successive, en direction longitudinale, des sections auto-adhésives faisant office de réservoir de substance active,

- couper par poinçonnage la couche dorsale,
- éliminer les rognures de la couche dorsale,
- couper ensuite la couche de protection dans les espaces intermédiaires ménagés entre les sections faisant office de réservoir de substance active.

21. Système thérapeutique transdermique selon l'une quelconque des revendications 1 à 19 pour faire office d'emplâtre à utiliser pendant plusieurs jours, en particulier pour le traitement de douleurs ou de la toxicomanie.

Fig.1

Fig.2